(19)

**Europäisches Patentamt**
**European Patent Office**
**Office européen des brevets**

(11) **EP 3 001 946 A1**

(12) **EUROPEAN PATENT APPLICATION**

(43) Date of publication:
**06.04.2016 Bulletin 2016/14**

(51) Int Cl.:
*A61B 5/00* [(2006.01)]     *A61B 5/024* [(2006.01)]
*A61B 5/026* [(2006.01)]     *A61B 5/0295* [(2006.01)]

(21) Application number: **14003368.9**

(22) Date of filing: **30.09.2014**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **BCB Informática y Control SL**
**28015 Madrid (ES)**

(72) Inventors:
• **Royo Royo, Santiago**
**08755 Castellbisbal (Barcelona) (ES)**

• **Azcona Guerrero, Francisco J.**
**08222 Terrassa (Barcelona) (ES)**
• **Jha, Ajit**
**08222 Terrassa (Barcelona) (ES)**
• **Bezares DEL Cueto, Javier**
**28015 Madrid (Madrid) (ES)**

(74) Representative: **Naranjo Marcos, Maria Antonia et al**
**Paseo de la Habana 200**
**ES-28036 Madrid (ES)**

(54) **Method, device and computer programs for measuring a fetal arterial pulse wave**

(57)     The method comprising: immobilizing with a fastener element (120) the relative motion of a part of the body (110) of a pregnant patient; pointing, with a laser device (130), to that part of the body (110) immobilized, measuring a first arterial pulse wave on the basis of an optical feedback interferometry technique; identifying from the measured first arterial pulse wave at least an anomaly (300); analyzing the identified anomaly (300) and further extracting depending on the result of said analysis, from a set of information representative of the first arterial pulse wave measured, a second arterial pulse wave associated to the first arterial pulse wave by using a set of signal processing algorithms, wherein the second arterial pulse wave corresponds to the arterial pulse wave of a fetus.

Fig. 3

EP 3 001 946 A1

**Description**

Field of the invention

The present invention generally relates to signal processing of biomedical signals. In particular, the invention relates to a method, a device and computer programs for measuring the arterial pulse wave of a fetus from the arterial pulse wave of a pregnant patient.

Background of the invention

The development of different non-invasive and non-contact methods in the last years for the acquisition of biomedical signals, such as cardiovascular signals, has increased the quality of patient care. Known techniques for biomedical signal measurement are for instance techniques based on optical sensors because of their simple construction, resolution, bandwidth, and low cost. Moreover, optical methods, in particular, have been assessed as a suitable solution for the measurement of different cardiovascular signals such as the cardiac rhythm and the arterial pulse wave or APW. The arterial pulse wave produced by the heart beat is propagated throughout the circulatory system, giving rise to an amplitude vibration (which is known as the pulse) that can be perceived with greater or lesser intensity in different parts of the body. A direct relation between said vibration and the human electrocardiogram is well known.

In F.J. Azcona et.al. "A new method for the acquisition of arterial pulse wave using self-mixing interferometry", Optics & Laser Technology 63 (2014) 98-104. 2014 Elsevier Ltd., of the same inventors of the present invention, it is disclosed a technique based on self-mixing interferometry as a method for the acquisition and reconstruction of the arterial pulse wave of a living being. In said article, a modification of the classic fringe counting reconstruction algorithm is proposed to deal with some of the problems caused by biological tissue surface roughness, therefore allowing a reconstruction of the arterial displacement with a resolution of 400nm. The traits of the arterial pulse wave were retrieved with high detail, allowing the interpretation by a skilled practitioner.

Currently the measure of the arterial pulse wave of a fetus, that is, the detection and reconstruction of the fetus-induced arterial pressure wave is only possible indirectly by ultrasound. Such method uses very bulky, and expensive, medical equipment, which is only available at major medical settings. Furthermore, the reliability of the measurement needs improvement, according to practitioners.

It is desirable to provide, therefore, a non-invasive and low cost method able to measure the fetal arterial pulse wave, in a way that maximizes chance for diagnose certain diseases of the fetus, as well as adding a new analysis element in known pathologies or contributing with novel means to the monitoring of fetal cardiovascular disorders.

Description of the invention

The inventors have developed a method by departing from the measure of the arterial pulse wave disclosed in the cited article. The object of this invention is based on that method, and obtains in addition to the arterial wave pulse of a pregnant patient, the measure of the associated fetal arterial pulse wave, which is known as being superimposed to the pressure wave of the mother but never previously detected from said first measure of the arterial wave pulse.

To that end, according to a first aspect there is provided a method for measuring a fetal arterial pulse wave which comprises measuring a first arterial pulse wave of a patient, and then further comprising;

-    identifying at least an anomaly in said measured first arterial pulse wave,
-    analyzing the identified anomaly, and
-    extracting depending on the result of said analysis, from a set of information representative of the first arterial pulse wave an associated second arterial pulse wave, which corresponds to the arterial pulse wave of a fetus, by using a set of signal processing algorithms executed by a processor.

By an anomaly, it has to be understood any amplitude changes in the arterial pulse wave of the mother which deviate from the conventional arterial pulse wave of a non-pregnant patient.

In accordance with an embodiment, the set of signal processing algorithms used is based on Wavelet analysis. Alternatively, in accordance to another embodiment, the set of signal processing algorithms used is based on the analysis of a mathematical adjustment over the measured first arterial pulse wave.

The first arterial pulse wave of a patient is measured according to the teaching from the cited article, using a fastener element that immobilizes the relative motion of a part of the body of the patient, and by pointing, with a laser device such as a laser diode, or another types of laser such as a surface emitting laser, an edge emitting laser, a Fabry-Perot laser, a vertical-cavity surface emitting laser (VCSEL), a distributed feedback laser (DFB) or a Distributed Bragg Reflector laser (DBR), among others, to that part of the body immobilized. The measuring of the first arterial pulse wave is preferably done on the basis of an optical feedback interferometry technique.

The immobilized part of the body may be a finger of the patient and the laser device may point to a nail of the immobilized finger. In an alternative the immobilized part may be for instance a wrist of the patient. In this case, the laser device may point to a vein of the immobilized wrist, because is the part of the body where better signal is obtained, however this is not limitative being other immobilized surfaces of the body exposed to a laser radiation also possible.

According to a second aspect there is provided a device for measuring a fetal arterial pulse wave, that includes a fastener element that immobilizes the relative motion of a part of the body of a patient, and a laser device such as a laser diode, among others, using an optical feedback interferometry technique, and that points to that part of the body immobilized for measuring a first arterial pulse wave.

The device of the second aspect of this invention characteristically further comprises processing means, including at least a processor that executes a set of signal processing algorithms aimed to identify at least an anomaly from the first arterial pulse wave measured and to analyze the identified anomaly and extract from a set of information representative of the first arterial pulse wave an associated second arterial pulse wave that corresponds to the arterial pulse wave of a fetus.

The processing means may be located in the device or, alternatively, may be remote to the device. Moreover, a part of the processing means may be located in the device and the other part may be remote to the device.

In accordance to an embodiment, the fastener element comprises a small surround housing or member surrounding a part of the patient body to be immobilized, wherein the part of the patient's body to immobilize is one of the fingers of the patient, as far it is the part where a better signal is obtained.

In accordance to another embodiment, the fastener element comprises a band, wherein the part of the patient's body to immobilize is one of the wrists of the patient which is surrounded and immobilized using the band.

Such embodiments should be considered as non limitative, as alternative fastener element arrangements for measuring the arterial pulse wave are possible.

Other arrangements of embodiments of the invention that are disclosed herein include software programs to perform the method embodiment steps and operations summarized above and disclosed in detail below. More particularly, a computer program product is one embodiment that has a computer-readable medium including computer program instructions encoded thereon that when executed on at least one processor in a computer system causes the processor to perform the operations indicated herein as embodiments of the invention.

Brief Description of the Drawings

The previous and other advantages and features will be more fully understood from the following detailed description of embodiments, with reference to the attached drawings, which must be considered in an illustrative and non-limiting manner, in which:

Figure 1 is a schematic setup for the measurement of arterial pulse wave according to an embodiment of the present invention. In this case the arterial pulse wave is measured by immobilizing a finger of the patient.

Figure 2 is a schematic setup for the measurement of arterial pulse wave according to another embodiment of the present invention. In this case the arterial pulse wave is measured by immobilizing a wrist of the patient.

Figure 3 illustrates a set of experimental results obtained by measuring the arterial pulse wave of a healthy adult woman, 38 weeks pregnant, according to an embodiment of the present invention.

Figure 4a illustrates an average function of the arterial pulse wave measured in Figure 3.

Figure 4b illustrates the scalogram of the arterial pulse wave measured in Figure 3 showing the percentage of energy at each frequency of the signal at each point of the pulse wave measured

Detailed Description of Several Embodiments

Present invention proposes from the data obtained by the teachings of cited article (measure of the arterial pulse wave of a living being) further obtaining, in the case of a female pregnant being to measure a fetal arterial pulse wave. Therefore, present invention from a first arterial pulse wave measure of a pregnant patient (the mother) is able to detect and reconstruct an associated second arterial pulse wave (the fetus-induced arterial pressure wave). For that purpose, present invention uses a set of signal processing algorithms executed by a processor of a computer system (not illustrated), preferably, as non-limiting examples, said algorithms are based on wavelets or on a mathematical adjustment of the measured arterial pulse wave of the mother.

Wavelet transformation is a method of investigating time frequency details of signals whose frequency content varies with time for e.g. non-stationary signals. Since OFI signals are dynamic in nature, so wavelet transform is one of the suited candidates to analyze it and draw important parameters related to target vibration. Wavelets are small waves that are localized both in time and in frequency. The wavelet transformation of a continuous signal x(t) is given by

$$W(a, b) = \frac{1}{\sqrt{a}} \int_{-\infty}^{+\infty} x(t)\gamma(\frac{t-b}{a})dt \quad (1)$$

$W(a, b)$ is the coefficient of wavelet transformation. $a$ and $b$ are scaling and translation parameters. Scaling parameter $a$ is in inverse relation to the frequency component of the analyzed signal x(t), thus giving frequency information of the signal and $b$ gives time information when said frequency component is generated. The physical interpretation of eq. (1) is that wavelet coefficients $W(a, b)$ are obtained by correlating the scaled and shifted version of the mother wavelet $\gamma(\frac{t-b}{a})$ with the signal to be analyzed (x(t)). For analyzing sine or cosine-like signals, the most commonly wavelet used is the Morlet wavelet. Further analysis of the signal using the Morlet wavelet results in complex wavelet coefficients from where amplitude and phase information of the analyzed signal can be extracted. The Morlet wavelet is defined in time domain as

$$\gamma(t) = \exp(j\omega_0 t)\exp\left(\frac{-t^2}{2}\right) \quad (2)$$

On another hand, a mathematical fitting of the pulse shape to some predefined function shape may be implemented. Typically, this involves measuring several pulse shapes to obtain an average shape which may be used as reference, and then setting a desired function shape with a number of parameters which, when fixed, enable the function to resemble closely the measured pulse shape. The quality of such adjustment is typically measured using the standard deviation of the differences amongst the original pulse shape and the desired data (called the residuals of the fitting). Several control software programs implement different types of advanced fitting algorithms and can be used with this purpose.

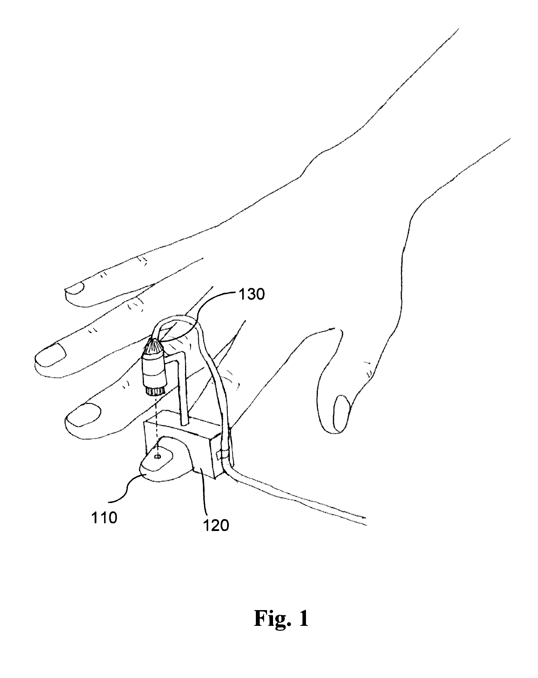

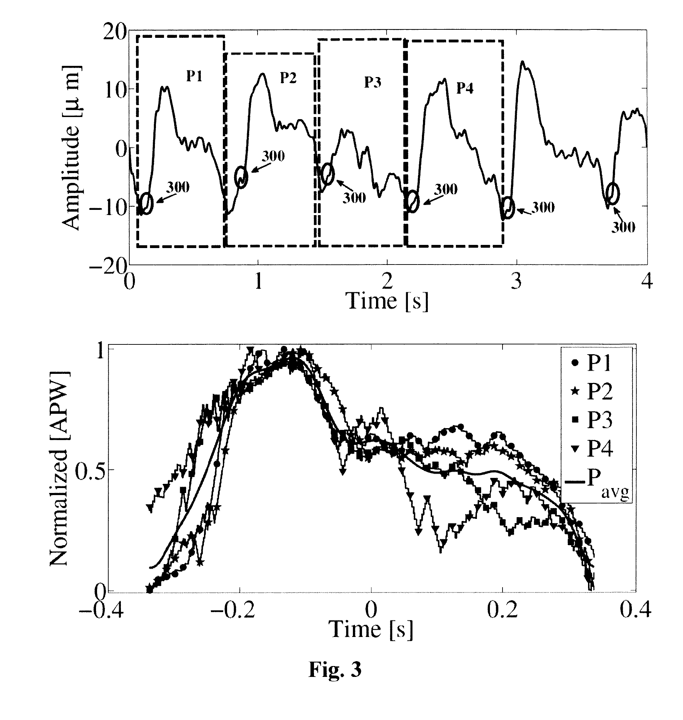

With reference to Fig. 1 it is illustrated an embodiment of the present invention for measuring a fetal arterial pulse wave. In this case, the fastener element 120 comprises a small surround housing and the part of the patient's body 110 immobilized is one of the fingers of the patient. When the laser device 130, which preferably uses an optical feedback interferometry technique, points to the immobilized part of the patient's body 110 the arterial pulse wave of the patient (the mother) is measured. Once the arterial pulse wave of the mother is measured, an anomaly (300 as can be seen from Fig. 3) thereof is identified and further analyzed to extract the arterial pulse wave of the fetus.

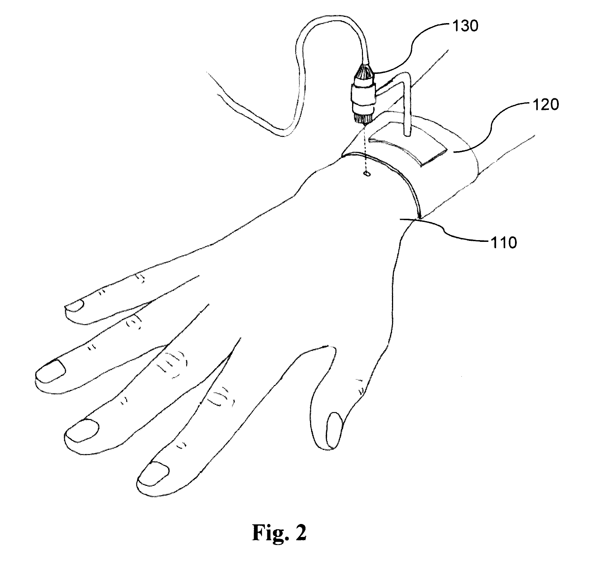

Fig. 2 illustrates another embodiment of the present invention for measuring a fetal arterial pulse wave. In this case, the fastener element 120 comprises a band and the part of the patient's body 110 immobilized is a wrist of the patient.

The fastener element 120, according to other embodiments, not illustrated, may also comprise a band or tape that can be located around the chest of the patient, or even on the ankle. In all cases the issue is to immobilize the relative motion of the part of the body where the fastener element 120 is located.

The laser device 130 preferably is a laser diode, monomode or multimode. Other types of laser devices 130 however are also possible, for instance a surface emitting laser, an edge emitting laser, a Fabry-Perot laser, a vertical-cavity surface emitting laser (VCSEL), a distributed feedback laser (DFB) or a Distributed Bragg Reflector laser (DBR), etc.

Fig. 3 shows the experimental results obtained of the measure of the arterial pulse wave of a healthy adult woman which was 38 weeks pregnant. Fig. 3 shows the reconstruction of a series of five pulses, showing the typical behavior of an arterial pulse wave. The signal, as showed in the figure, contains the combination of the arterial pulse wave of the mother and of the fetus. As detailed before, present invention to detect and reconstruct the fetus-induced arterial pressure wave uses a set of signal processing algorithms which are preferably based on the analysis of wavelets or on a mathematical adjustment of the measured arterial pulse wave of the mother.

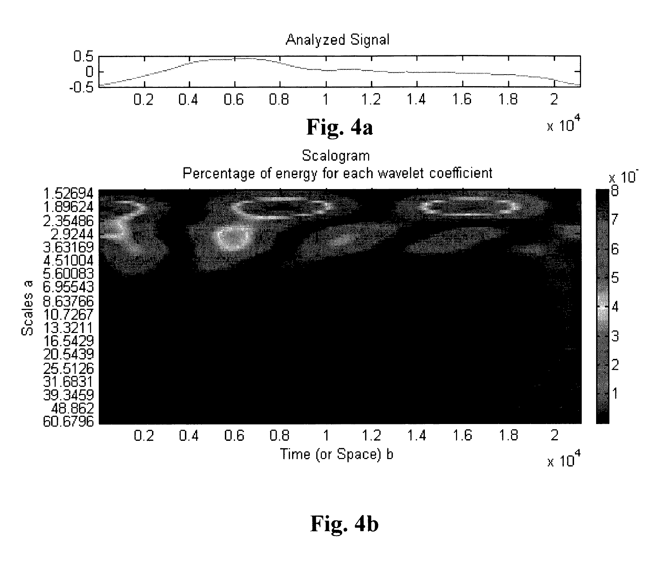

The results of the mathematical adjustment of a double Gaussian average of the arterial pulse wave function, as well as the difference between the adjustment and the original measure, are illustrated in Fig. 4a. It can be appreciated that the adjustment residual (which contains noise acquisition elements together with the fetal arterial pulse wave) presents maximum amplitude between $2\mu m$ and $4\mu m$, and approximately a double frequency of the mother, being data consistent with those expected for the fetal arterial pulse wave.

On another hand, the analysis of the average arterial pulse wave illustrated in Fig. 3 using wavelet functions is illustrated in Fig. 4b, where under the arterial pulse wave analyzed it can be seen the existence of another signal of lower intensity and dual-frequency in the representation of the scalogram of the wavelet function, which represents the percentage of energy present in each of the frequencies and signal moments. The existence of a dual-frequency signal becomes evident in the existence of a maximum at the frequency approximately twice the frequency of the maximum principal.

The exemplary computer system includes the processor and may at least also include a main memory (e.g., read-only memory (ROM), flash memory, dynamic random access memory (DRAM) such as synchronous DRAM (SDRAM) or Rambus DRAM (RDRAM), etc.), a static memory (e.g., flash memory, static random access memory (SRAM), etc.), and a data storage device.

Additionally, the software programs included as part of the invention may be embodied in a computer program product that includes a computer useable medium. For example, such a computer usable medium can include a readable memory device, such as a hard drive device, a flash memory device, a CD-ROM, a DVD/ROM, or a computer diskette, having computer readable program code segments stored thereon. The computer readable medium can also include a communications link, either optical, wired, or wireless, having program code segments carried thereon as digital or analog signals.

While certain embodiments have been described, these embodiments have been presented by way of example only, and are not intended to limit the scope of the protection. Indeed, the novel methods and apparatuses described herein may be embodied in a variety of other forms. The accompanying claims and their equivalents are intended to cover such forms or modifications as would fall within the scope and spirit of the protection.

**Claims**

1. A method for measuring a fetal arterial pulse wave, the method comprising:

   - immobilizing with a fastener element (120) the relative motion of a part of the body (110) of a pregnant patient;
   - pointing, with a laser device (130), to that part of the body (110) immobilized, and
   - measuring a first arterial pulse wave on the basis of an optical feedback interferometry technique,
   the method being **characterized in that** further comprising:
   - identifying from the measured first arterial pulse wave at least an anomaly (300);
   - analyzing the identified anomaly (300) and further extracting depending on the result of said analysis, from a set of information representative of the first arterial pulse wave measured, a second arterial pulse wave associated to the first arterial pulse wave by using a set of signal processing algorithms executed by a processor, wherein the second arterial pulse wave corresponds to the arterial pulse wave of a fetus.

2. The method of claim 1, wherein the set of signal processing algorithms used is based on Wavelet analysis.

3. The method of claim 1, wherein the set of signal processing algorithms used is based on the analysis of a mathematical adjustment over the measured first arterial pulse wave.

4. The method of any of previous claims, wherein the part of the body immobilized (110) comprises a finger of the patient, the laser device (130) pointing to a nail of the immobilized finger.

5. The method of any of previous claims 1 to 3, wherein the part of the body (110) immobilized comprises a wrist of the patient, the laser device (130) pointing at least to a vein of the immobilized wrist.

6. The method of any of previous claims, wherein the laser device (130) comprises one of a laser diode, a surface emitting laser, an edge emitting laser, a Fabry-Perot laser, a vertical-cavity surface emitting laser, a distributed feedback laser or a Distributed Bragg Reflector laser.

7. A device for measuring a fetal arterial pulse wave, comprises:

   - a fastener element (120) that immobilizes the relative motion of a part of the body (110) of a patient, and
   - a laser device (130) using an optical feedback interferometry technique that points to said part of the body (110) immobilized for measuring a first arterial pulse wave,
   the system being **characterized in that** it further comprises processing means to identify at least an anomaly (300) from the measured first arterial pulse wave and to analyze the identified anomaly (300) and further extract, from a set of information representative of the first arterial pulse wave, a second arterial pulse wave associated to the first arterial pulse wave, wherein the processing means includes at least a processor that executes a set of signal processing algorithms and wherein the second arterial pulse wave corresponds to the arterial pulse wave of a fetus.

8. The device of claim 7, wherein a part of the processing means is located in the device and a part is remote to the device.

9. The device of claim 7, wherein the processing means are located in the device.

10. The device of claim 7, wherein the processing means are remote to the device.

11. The device of any of previous claims 7 to 10, wherein the laser device (130) comprises one of a laser diode, a surface emitting laser, an edge emitting laser, a Fabry-Perot laser, a vertical-cavity surface emitting laser, a distributed feedback laser or a Distributed Bragg Reflector laser.

12. The device of any of previous claims 7 to 11, wherein the fastener element (120) comprises a small surround housing, being the part of the patient's body (110) to immobilize one of the fingers of the patient.

13. The device of any of previous claims 7 to 11, wherein the fastener element (120) comprises a band, being the part of the patient's body (110) to immobilize at least one of the wrists of the patient which is surrounded and immobilized using the band.

14. A computer program which when executing on a computer or computer network performs the steps of any one of claims 1, 2 or 3.

15. The computer program of claim 14, stored on a computer-readable medium.

**Fig. 1**

**Fig. 2**

**Fig. 3**

Analyzed Signal

**Fig. 4a**

x 10⁴

Scalogram
Percentage of energy for each wavelet coefficient

**Fig. 4b**

Europäisches
Patentamt

European
Patent Office

Office européen
des brevets

# EUROPEAN SEARCH REPORT

Application Number

EP 14 00 3368

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ARASANZ A ET AL: "A new method for the acquisition of arterial pulse wave using self-mixing interferometry", OPTICS AND LASER TECHNOLOGY, vol. 63, 25 April 2014 (2014-04-25), pages 98-104, XP029028841, online ISSN: 0030-3992, DOI: 10.1016/J.OPTLASTEC.2014.04.004 * abstract * | 1-15 | INV. A61B5/00 A61B5/024 A61B5/026 A61B5/0295 |
| A | US 2004/116789 A1 (BOAS DAVID ALAN [US] ET AL) 17 June 2004 (2004-06-17) * paragraphs [0004] - [0006] * | 1-15 | |
| A | US 2008/269625 A1 (HALPERIN AVNER [IL] ET AL) 30 October 2008 (2008-10-30) * paragraphs [0234], [0235], [0627] - [0645] * | 1-15 | |
| A | US 2011/218413 A1 (WANG YIXIANG [US] ET AL) 8 September 2011 (2011-09-08) * paragraphs [0036] - [0048] * | 1-15 | TECHNICAL FIELDS SEARCHED (IPC) |
| | | | A61B |

The present search report has been drawn up for all claims

| Place of search | Date of completion of the search | Examiner |
|---|---|---|
| Berlin | 5 March 2015 | Trachterna, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 001 946 A1**

## ANNEX TO THE EUROPEAN SEARCH REPORT
## ON EUROPEAN PATENT APPLICATION NO.

EP 14 00 3368

05-03-2015

| Patent document cited in search report | | Publication date | Patent family member(s) | | Publication date |
|---|---|---|---|---|---|
| US 2004116789 | A1 | 17-06-2004 | AU | 3459001 A | 07-08-2001 |
| | | | CA | 2397840 A1 | 02-08-2001 |
| | | | EP | 1251770 A1 | 30-10-2002 |
| | | | US | 2004116789 A1 | 17-06-2004 |
| | | | WO | 0154573 A1 | 02-08-2001 |
| US 2008269625 | A1 | 30-10-2008 | US | 2008269625 A1 | 30-10-2008 |
| | | | US | 2013137998 A1 | 30-05-2013 |
| | | | US | 2013144178 A1 | 06-06-2013 |
| | | | US | 2014012099 A1 | 09-01-2014 |
| | | | US | 2014207204 A1 | 24-07-2014 |
| US 2011218413 | A1 | 08-09-2011 | NONE | | |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- A new method for the acquisition of arterial pulse wave using self-mixing interferometry. **F.J. AZCONA.** Optics & Laser Technology. Elsevier Ltd, 2014, vol. 63, 98-104